# EUROPEAN PATENT SPECIFICATION

(11) **EP 3 151 742 B1**
(45) Date of publication and mention of the grant of the patent: **28.02.2024**
(21) Application number: 15806601.9
(22) Date of filing: 09.06.2015
(51) Int. Cl.: A61B 5/00, G06F 3/01, A61B 5/291

(54) **BRAIN-COMPUTER INTERFACE HEADSET**
HAUBE MIT GEHIRN-COMPUTER-SCHNITTSTELLE
CASQUE D'INTERFACE CERVEAU-ORDINATEUR

(30) Priority: 09.06.2014 US 201462009488 P
(43) Date of publication of application: 12.04.2017
(73) Proprietor: Neurolutions, Inc., Clayton, Missouri 63105 (US)
(72) Inventor: LEUTHARDT, Eric C., St. Louis, Missouri 63130 (US); MORAN, Daniel W., Ballwin, Missouri 63021 (US)
(74) Representative: Fish & Richardson P.C.
(86) International application number: PCT/US2015/034841
(87) International publication number: WO 2015/191538

(56) References cited:
- WO-A1-2008/119031
- WO-A2-2007/109745
- KR-A- 20110 061 733
- US-A- 4 486 630
- US-A- 6 097 981
- US-A1- 2005 107 716
- US-A1- 2011 015 503
- US-A1- 2013 172 721
- US-A1- 2013 303 874
- US-A1- 2014 051 044
- US-B1- 6 381 481
- US-B1- 6 381 481
- US-B2- 8 103 328
- US-B2- 8 165 684

## Description

### FIELD OF THE INVENTION

This specification relates to components of brain-computer interface (BCI) systems, namely, electroencephalography (EEG) headsets that can be used as part of a BCI system.

### BACKGROUND

BCI technology involves determining a person's intentions by acquiring and interpreting the person's brain signals, and executing the intended tasks using a computer system. One example application of BCI technologies is the control of a cursor on a computer screen. There are many others.

Brain cells communicate with each other by producing tiny electrical signals. The goal of EEG is to noninvasively detect and quantify those tiny electrical signals. The electrical signals detected by EEG are then processed and interpreted by other BCI system components to determine the intentions of the person.

Some amounts of the tiny electrical signals produced by the brain cells are transferred to the scalp of the person. EEG is performed by placing multiple electrodes in contact with the scalp to receive those electrical signals present on the scalp. The accuracy of EEG can be affected by a number of factors including, but not limited to, electrode placement locations, integrity of electrode-to-scalp contact, electrical interference, and others.

US 6381481 B1 describes an EEG electrode locator headgear.

WO 2007/109745 A2 describes an electrode headset and electrodes that can be mounted therein.

US 2014/0051044 A1 describes methods for obtaining electroencephalographic (EEG) data from a subject via a device comprising two or more independently adjustable bands.
KR20110061733A discloses a helmet provided with EEG electrodes. The EEG electrodes are wired to a control unit using conductive fibers that are inserted in the fabric structure of the helmet. The helmet is provided with a chin strap containing conductive fibers used to detect whether the chin strap is fastened or unfastened.

### SUMMARY

**The** invention is defined in the claims.

In one implementation, an EEG headset apparatus for detecting brain signal information from a human's head includes a casing with an outer surface and an inner surface, a conductive EMI shield material, and a conductive wire electrically coupled to the conductive EMI shield and configured to conduct energy received from the conductive EMI shield to a region of the human that is distinct from sites recording the brain signal information. The casing's shape defines a concaved interior region configured to receive a portion of the head. The casing defines a plurality of electrode openings. Each electrode opening of the plurality of electrode openings comprises a hole extending between the outer surface and the inner surface. The conductive EMI shield material is disposed either (i) on the inner or outer surfaces of the casing, (ii) in a wall of the casing, or (iii) within the interior region defined by the casing. The conductive EMI shield is configured to operate as an EMI shield. A conductive periphery is disposed around at least around portions of an inner periphery of the casing, wherein the inner periphery of the casing interfaces with the skin of the human and the conductive periphery is in electrical communication with the EMI shield, and in physical contact with the skin of the human. The EEG headset apparatus further comprises a chin strap coupled to the casing. The chin strap includes the conductive wire and a conductive contact clement that contacts the skin of the human beneath the chin or the chin strap is made of an electrically conductive material and is in electrical communication with the conductive EMI shield material (124) and the skin of the user.

Such an EEG headset apparatus may optionally include one or more of the following features. The EEG headset apparatus may include one or more electrode assemblies, wherein each electrode assembly of the one or more electrode assemblies is configured to be disposed within one electrode opening of the plurality of electrode openings, and wherein each electrode assembly is configured to receive the brain signal information from a surface of the human's head. The one or more electrode assemblies may be configured to be physically repositionable in relation to respective electrode openings of the casing. The one or more electrode assemblies may be configured to be slidably and pivotably repositionable in relation to respective electrode openings of the casing. At least a portion of the conductive wire may be coupled with the chin strap. The EEG headset apparatus may further comprise controller circuitry including one or more microprocessors, wherein the controller circuitry is configured to receive the brain signal information. The EEG headset apparatus may further comprise a user interface in electrical communication with the controller circuitry, wherein the user interface comprises one or more elements by which (i) user inputs can be provided to the controller circuitry or (ii) outputs can be provided from the controller circuitry. The EEG headset apparatus may further comprise a communications interface in electrical communication with the controller circuitry, wherein the communications interface is configured to facilitate communications between the controller circuitry and an external computing device. The communications interface may be a wireless communications interface.

In another implementation, a brain-computer interface system can includes a helmet-like apparatus. The helmet-like apparatus can comprise and EEG headset apparatus as described above.

Particular embodiments of the subject matter described in this document can be implemented to realize one or more of the following advantages. In some embodiments, the EEG headsets provided herein are readily adaptable for effective use with a variety of head shapes and sizes. Accordingly, the EEG headsets can be fitted for a person, and the person can repeatedly use the EEG headset without further assistance from a clinician. As such, the EEG headsets provided herein are well-suited for home use. In some embodiments, the EEG headsets are configured for single-handed use. Consequently, in some cases people who are physically unable to use two hands/arms may nevertheless be able to don, operate, and doff the EEG headsets provided herein. In some embodiments, the EEG headsets provided herein are shielded against electrical interference from ambient electrical fields. Such shielding can eliminate some inaccuracies in the EEG results. Further, in some embodiments the EEG headsets are configured to be resistive to inaccuracies induced from the activity of muscle movements, such as jaw movements, for example. Various types of additional sensors are included in some embodiments of the EEG headsets provided herein. Accordingly, some embodiments of the EEG headsets can provide additional functionalities beyond EEG. A graphical user interface (GUI) is included in some embodiments of the EEG headsets provided herein. The GUI may enhance a user's interactions with the EEG headsets. The EEG headsets provided herein are configured with features that facilitate user-friendly, accurate, and repeatable EEG performance.

Unless otherwise defined, all technical and scientific terms used herein have the same meaning as commonly understood by one of ordinary skill in the art to which this invention pertains. Although materials similar or equivalent to those described herein can be used to practice the invention, suitable materials are described herein. In addition, the materials, methods, and examples are illustrative only and not intended to be limiting.

The details of one or more embodiments of the invention are set forth in the accompanying drawings and the description herein. Other features, objects, and advantages of the invention will be apparent from the description and drawings, and from the claims.

### BRIEF DESCRIPTION OF THE DRAWINGS

FIG. 1 is a diagram of an example EEG headset and BCI system, shown in operation by a user.
FIG. 2 is a side view of the example EEG headset of FIG. 1.
FIG. 3A is a cross-sectional side view of the example EEG headset of FIG. 1 being worn by a user.
FIG. 3B is a portion of the cross-sectional side view of the example EEG headset as shown in FIG. 3A including an example EEG electrode.
FIG. 3C is a schematic depiction illustrating the adjustability of EEG headset electrodes in accordance with some embodiments.
FIG. 4 is a schematic diagram of computing devices that can be used to implement the systems and techniques described herein.

Like reference numbers and designations in the various drawings indicate like elements.

### DETAILED DESCRIPTION

This specification generally describes EEG headsets that can be used in BCI systems. The EEG headsets described herein provide user convenience and EEG accuracy. For example, this specification describes EEG headsets that are readily customizable for effective use with a wide variety of head shapes and sizes. An EEG headset with a customized fit can provide convenient repeatable electrode-to-scalp contact for a particular user. Further, this specification describes EEG headsets with integrated electromagnetic shielding for reducing electromagnetic interference (EMI) and improving EEG accuracy.

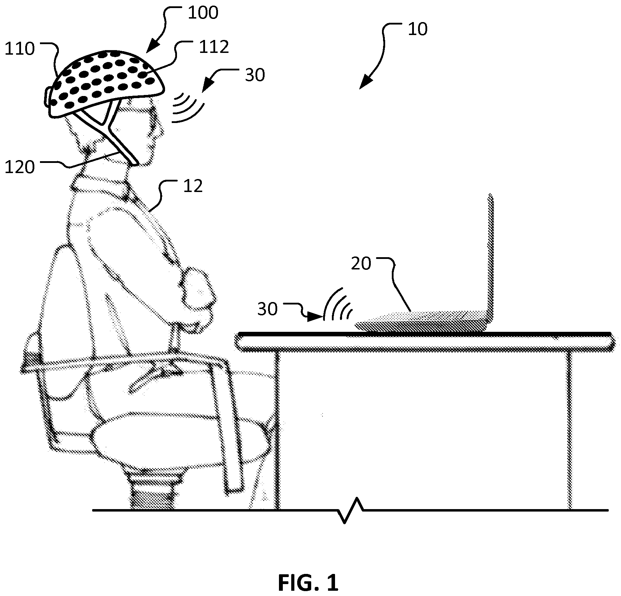

One example implementation, shown in **FIG. 1****,** is a BCI system 10 which is adapted for operation by a user 12. Generally, the system 10 includes: (i) a wearable EEG headset 100 and (ii) a computing device 20. In the depicted implementation, the EEG headset 100 and the computing device 20 wirelessly communicate with each other as depicted by wireless signal symbols 30. In some embodiments, as an alternative to the wireless communication or in addition to the wireless communication, one or more communication cables (not shown) are used to interconnect the EEG headset 100 and the computing device 20 to facilitate data communications therebetween.

The computing device 20 is generally adapted to receive wirelessly transmitted signals 30, sent by the EEG headset 100, containing information about the user's 12 brain signals acquired by the EEG headset 100. The computing device 20 is configured to process those received signals 30 to determine user's 12 intentions. In some embodiments, the computing device 20 is configured to take actions in accordance with the determined user's 12 intentions. The computing device 20 can be any of a variety of different types of computing devices. For example, the computing device 20 can be, but is not limited to, the following types of computing devices: laptop computer, desktop computer, cell phone, smart phone, PDA, tablet computer, music player, wearable computer, e-book reader, server system, or other processing device and combinations of devices.

In some implementations of the BCI system 10, certain operations or parts of certain operations may be performed at a server system, including a cloud-based server system, rather than completely on a client computing device such as the computing device 20. However, in other embodiments, all or substantially all of the operations of the BCI system 10 may be performed on the computing device 20.

In some implementations, the computing device 20 may also include one or more additional components such as a mouse, trackball, touchpad, joystick, touchscreen, auditory input and output devices, tactile input and output devices, wireless communication interface devices, and the like, and combinations of such devices. The computing device 20 can be connected to any form or medium of digital data communication (e.g., a communication network). Non-limiting examples of communication networks include a local area network (LAN), a wide area network (WAN), and the Internet.

In the depicted implementation, computing device 20 and the EEG headset 100 perform two-way communication via wireless signals 30. Such wireless communication may occur using various wireless technologies including, but not limited to, radio-frequency, Bluetooth, WiFi, NFC, IR, Bluetooth low energy, ANT+, and the like.

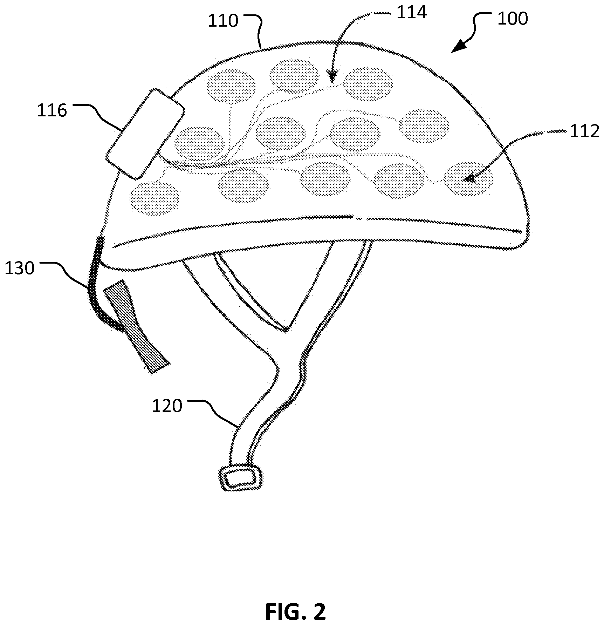

Briefly, the EEG headset 100 includes: (i) an electrode support system 110, which in this example is configured like a helmet 110 for holding several surface electrodes that acquire EEG brain signals from multiple different and distributed surface locations on the user's 12 skin adjacent the brain, and (ii) a chinstrap 120. In this example, the chinstrap 120 extends between both sides of the helmet 110 and under the chin of the user 12. By using such an arrangement, the chinstrap 120 can provide a normal force between the helmet 110 and head of the user 12, and more particularly between the electrodes of the helmet 110 and the head of the user 12. In some embodiments, a flexible occipital buttress (e.g., refer to FIG. 2) can be alternatively or additionally included to secure the helmet 110 to the head of the user 12.

**The** helmet 110 includes multiple electrode locations 112. As will be described more fully below, the electrode locations 112 may receive an adjustable electrode assembly therein. When the helmet 110 is configured for use, at least some of the electrode locations 112, but not necessarily all of the electrode locations 112, contain electrode assemblies. Such electrode assemblies are individually positionable relative to helmet 110 to adapt to the particular head size and shape of the user 12. That is, the electrode assemblies can be individually adjusted so that the electrodes are conformed to the contoured topography of the user's 12 scalp. In that manner, accurate pickup of EEG brain signals from multiple different and distributed surface locations on the user's 12 scalp is facilitated by the EEG headset 100.

In some embodiments, when the fit of the EEG headset 100 has been customized for the user 12, the user 12 may repeatably use the EEG headset 100 without making further adjustments, or with few adjustments. That is, after the EEG headset 100 has been customized for the user 12, the user 12 may readily don the EEG headset 100 by merely placing the EEG headset 100 on the user's 12 head and installing the chinstrap 120 as shown. Therefore, the EEG headset 100 is designed to allow the BCI system 10 to be user-friendly and convenient for the user 12 to operate with minimal or no assistance from a clinician. As such, the BCI system 10 is well-suited for operation by the user 12 at home and/or at other locations that are remote from a healthcare facility. However, the adjustability of the helmet 110 also makes the BCI system 10 suitable for use at a healthcare facility where the helmet 110 can be efficiently readjusted as needed to fit multiple different patients.

In some implementations, the helmet 110 is initially fitted to the user 12 by a clinician. However, it is not necessary in all implementations for a clinician to provide the initial fitting of the helmet 110 to the user 12. In some implementations, the user 12 themselves can perform the fitting.

In some implementations, the user 12 can don the helmet 110 using a single hand. In some such implementations, the chinstrap 120 may include an elastic portion, a ratcheting mechanism, or be otherwise conveniently adjustable to facilitate one-handed adjustments.

Referring now to **FIG. 2****,** an example embodiment of the EEG headset 100 is shown in greater detail. The EEG headset 100 includes the helmet 110, the chinstrap 120, the multiple electrode locations 112, multiple wires 114, an electronics enclosure 116, and an optional occipital brace member 130. It should be understood that the depicted sites of the multiple electrode locations 112 are merely illustrative and the electrode locations 112 can be positioned anywhere in relation to the helmet 110.

**The** optional occipital brace member 130 can be mounted to the helmet 110 or to the chinstrap 120. In some embodiments, the occipital brace member 130 is physically adjustable such that a user can tighten and loosen the fit of the EEG headset 100 to the user's head by adjusting the orientation of the occipital brace member 130.

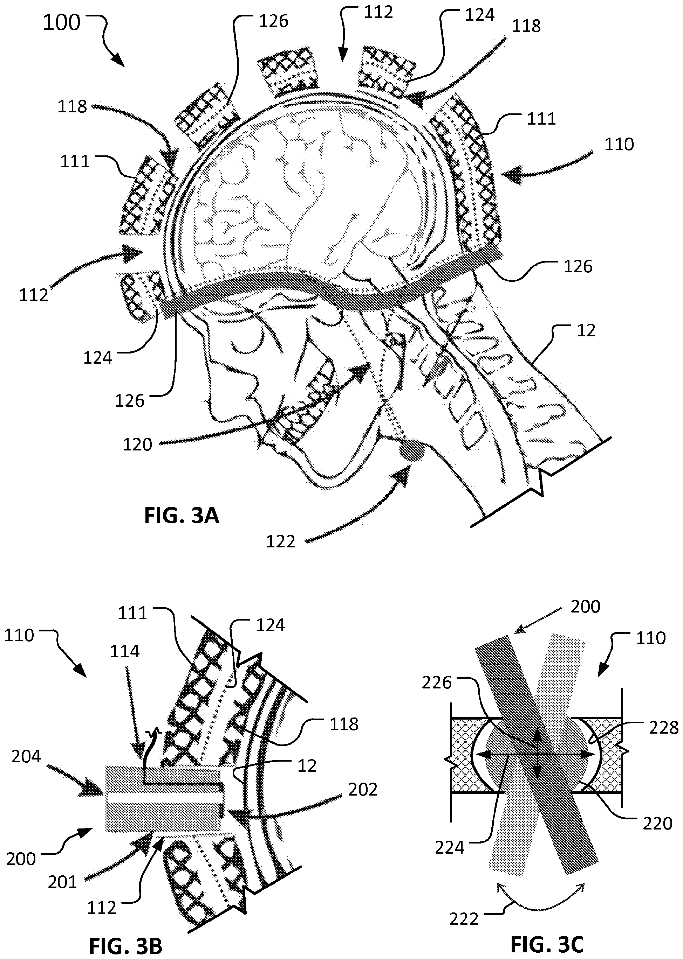

The wires 114 individually extend between the EEG electrodes (not shown) that are located in some or all of the electrode locations 112 and the electronics enclosure 116. The EEG signals detected by the EEG electrodes are carried by the wires 114 to the electrical controller circuitry located in the electronics enclosure 116. In the depicted implementation, the EEG electrodes are active electrodes. That is, the EEG electrodes include amplifiers in the proximity of the interface between the EEG electrodes and the skin. As such, with the EEG signals being amplified, the wires 114 can be routed from the EEG electrodes to the electronics enclosure 116 on or near the surface of the helmet 110 without incurring significant electromagnetic interference (EMI) effects. In some implementations, the wires 114 are shielded, either individually or by being located within an EMI shield of the helmet 110 (e.g., refer to FIGS. 3A and 3B). In some such implementations, passive electrodes (without the aforementioned amplifiers) are used. However, active electrodes may be used in some such implementations that include shielded wires 114.

While in the depicted embodiment the electronics enclosure 116 is located on the surface of the rear portion of the helmet 110, that location is not required in all embodiments. For example, in some embodiments the electronics enclosure 116 is located on the front of the helmet 110, or on the top of the helmet 110, or on other locations of the helmet 110. In some embodiments, the electronics enclosure 116 is embedded within the profile of the helmet 110, as opposed to being surface mounted as shown. In some embodiments, the contents of the electronics enclosure 116 are distributed within two or more electronics enclosures 116 that can be separated from each other in various locations on the helmet 110.

In some embodiments, the electronics enclosure 116 is releasably attachable to the helmet 110. Such an arrangement can conveniently allow a user to be able to handle to the electronics enclosure 116 while concurrently wearing the helmet 110. In some such embodiments the user can, for example, view a user interface on the electronics enclosure 116, and make selections and/or adjustments to the EEG headset 100 via the user interface while wearing the helmet 110.

The electronics enclosure 116 can contain various electronic components. For example, electronic components such as, but not limited to, the following can be included: one or more batteries, microprocessor(s), one or more types of memory devices, control circuitry, transceivers, antennae, gyroscopes, accelerometers, oximetry circuitry, electrode amplifiers, various kinds of connectors (e.g., USB ports, power supply ports, audio/video input and/or output ports, network connection ports, etc.), user interface elements (e.g., a graphical display, a touchscreen graphical display, a microphone to receive audio input from the user, a camera, audio speakers, indicator lights, buttons, keys, switches, tactile feedback devices, and the like). The one or more batteries can allow the EEG headset 100 to be portable (the batteries can provide power to the various components of the EEG headset 100, and may be recharged via an adapter or charging device (not shown here). In some embodiments, the batteries of the EEG headset 100 can be inductively recharged.

**The** EEG headset 100 includes the processing and controller circuitry to operate the EEG headset 100 in training modes, operational modes (e.g., rehabilitation sessions), calibration modes, communications modes, and so on. As such, the EEG headset 100 can include one or more central processing units, volatile memory such as random access memory (RAM), and non-volatile memory such as read-only memory (ROM) and/or various forms of programmable read-only memory (PROM) for the storage of software or firmware programs and operating parameters that may be periodically updated. In terms of software and/or firmware programs, the EEG headset 100 may include various programs that are stored in non-volatile memory that include executable program instructions that are executed by the processing and control circuitry to carry out the various processing functions. The non-volatile memory may also include information storage areas for operational parameter settings or other input information used during the operation of the EEG headset 100. The settings and other input information may be input by a user (or clinician), or may be transmitted to the EEG headset 100 from a remote system.

**As** mentioned briefly above, the EEG headset 100 can include various components for providing information to and receiving input from a user. The visual output display equipment, for example, may be a regular or touch screen display for providing visual prompts (e.g., graphics, instructions, etc.) or other sorts of information to the user and/or for receiving user input. The input devices, for example, may include one or more buttons for controlling (e.g., pausing, powering on/off, sending data, receiving data, changing modes, etc.) the EEG headset 100. For example, the input devices (e.g., buttons) may serve as soft keys alongside the display equipment and/or may be situated away from the display equipment. The audio output equipment (e.g., speakers), for example, may be used for providing auditory prompts (e.g., live or recorded spoken instructions, tones indicating success or error conditions, etc.). The audio input equipment (e.g., microphone), for example, may be used for receiving spoken input from the user (e.g., voice controls) and/or may serve with the audio output equipment for conducting a live communication session with a remote technician.

Referring now to **FIG. 3A****,** the EEG headset 100 is shown in partial cross-section on the head of the user 12. In this view, it can be seen that the helmet 110 includes a rigid shell 111, a conformable liner 118, an EMI shield 124, and a conductive periphery 126. The helmet 110 defines the aforementioned multiple electrode locations 112 and includes the chinstrap 120. The chinstrap 120 includes a conductive wire and a contact element 122 that contacts the user's 12 skin beneath the chin.

In some embodiments, the rigid shell 111 defines the outer profile of the helmet 110. The rigid shell 111 can be made of any of a variety of different materials such as polymers including, but not limited to, polycarbonate, polyvinyl chloride (PVC), polyethylene, polypropylene, polyurethane, polymethyl methacrylate, polystyrene, acrylonitrile butadiene styrene (ABS), polyethylene, polypropylene, polymide, and the like. In some embodiments, the rigid shell 111 is formed by being molded (e.g., injection molded).

The EMI shield 124 is disposed on or within the rigid shell 111. The EMI shield 124 is configured to function as a Faraday cage to reduce the negative impacts of EMI and radio frequency interference (RFI) on the fidelity of the EEG signals detected from the user 12. That is, such shielding may eliminate some inaccuracies in the EEG results by isolating the EEG electrode signals from ambient electrical noise. In some embodiments, the EMI shield 124 is made of a metal screen or mesh material. For example, a copper (or other conductive metal) screen or mesh is used in some embodiments. In some embodiments, the metal screen or mesh material is overmolded during the formation of the rigid shell 111. In some embodiments, the metal screen or mesh material is attached to the rigid shell 111 after the rigid shell 111 is formed.

In some embodiments, a conductive coating is applied to the rigid shell 111 to comprise the EMI shield 124. In some such embodiments, the conductive coating can include small metallic particles (e.g., copper or nickel) that are dispersed in a suitable carrier material. The dispersion can be sprayed, brushed, or otherwise coated onto the rigid shell 111. In some embodiments, the EMI shield 124 can comprise a combination of the metal screen or mesh material and the conductive coating(s). In particular embodiments, the EMI shield 124 is configured to be an active shield (e.g., a negative capacitance circuit).

In some embodiments, the aforementioned wires 114 (refer to FIG. 2) are substantially disposed within the protective Faraday cage environment provided by the EMI shield 124. In some such embodiments, passive EEG electrodes (without amplifiers near the electrodes) can be used, and the EMI shield 124 will substantially protect the wires 114 from the effects of EMI/RFI. Alternatively, in some embodiments the EEG electrodes used are active electrodes. That is, the EEG electrodes include amplifiers in the proximity of the interface between the EEG electrodes and the skin. In some such embodiments, the wires 114 can be routed from the EEG electrodes to the electronics enclosure 116 outside of the EMI shield 124 without incurring significant effects from EMI/RFI. In some embodiments, wires 114 from active EEG electrodes are routed within the EMI shield 124.

**The** helmet 110 can also include the conformable liner 118. The conformable liner 118 is disposed on at least some portions of the interior of the helmet 110. The conformable liner 118 is included to provide user 12 comfort, and to physically conform the helmet 110 to the contours of the scalp of the user 12. The conformable liner 118 can be, for example, a foam material, a gel encased in a flexible jacket material, or other suitable conformable materials. In some embodiments, portions of the conformable liner 118 may be removable from the helmet 110 and replaced with thicker or thinner portions of the conformable liner 118. In that fashion, the fit of the helmet 110 can be customized to the size and contours of the scalp of the user 12.

**The** multiple electrode locations 112 are defined by openings in the helmet 110 (including openings through the rigid shell 111, the EMI shield 124, and the conformable liner 118). In some embodiments, the helmet 110 may include about 20 to about 40 electrode locations 112, which may be located in any location on the helmet 110. In some embodiments, fewer than 20 or more than 40 electrode locations 112 are included in a helmet 110.

**As** previously described, when the helmet 110 is configured for use, at least some of the electrode locations 112, but not necessarily all of the electrode locations 112, contain electrode assemblies. In some implementations, when an electrode location 112 does not contain an electrode, a plug (not shown) is installed in the electrode location 112. Such plugs can be removably coupled within the electrode locations 112. In some embodiments, the plugs include the EMI shield 124 material that makes electrical contact with the EMI shield 124 of the helmet 110 so as to provide a continuous Faraday cage in the region of an electrode location 112 that does not contain an electrode.

**The** helmet 110 also includes the conductive periphery 126. In some embodiments, the conductive periphery 126 is disposed around at least portions of the inner periphery of the helmet 110, where the inner periphery of the helmet 110 interfaces with the skin of the user 12. The conductive periphery 126 is in electrical communication with the EMI shield 124, and in physical contact with the skin of the user 12. With such a configuration, EMI/RFI energy that is inducted to the EMI shield 124 can be conducted to the conductive periphery 126 where the EMI/RFI energy can be grounded to the skin of the user 12. In addition, the conductive periphery 126 can also ground out electromyographic activity (EMG) from, for example, jaw muscle movements in some circumstances. Such protection from the effects of EMG can also enhance the fidelity of the EEG electrode signals.

In some embodiments, the conductive periphery 126 is constructed as a compliant conductive strip of material that is attached around at least portions of the inner periphery of the helmet 110. In some embodiments, the conductive periphery 126 is adjustable in size, such as by an integral drawstring or by use of compliant materials, so that the conductive periphery 126 is customizable to fit the head of the user 12.

The helmet 110 also includes the chin strap 120 with its optional conductive contact element 122. The chin strap 120 is made of an electrically conductive material or includes an electrically conductive element therein. The chin strap 120 is in electrical communication with the EMI shield 124 and the conductive periphery 126. The chin strap 120 is also in electrical communication with the skin of the user 12. The chin strap 120 can thereby ground EMI/RFI energy from the EMI shield 124 to the skin of the user 12. Alternatively, or additionally, the contact element 122 can be used to ground EMI/RFI energy from the EMI shield 124 to the user's 12 skin beneath the chin. The contact element 122 can be configured to provide an effective electrical contact between the chin strap 120 and the user's 12 skin. For example, in some embodiments, a conductive gel material (or water, for example) can be included or applied to the contact element 122 to provide good electrical contact with the user's 12 skin. However, in some embodiments the contact element 122 can be used in a dry condition. In alternative embodiments, the contact element 122 can be located at other locations of the user's body (e.g., further away from the user's 12 head area).

Referring now to **FIG. 3B****,** the helmet 110 (shown in partial cross-section) includes the electrode locations 112 in which electrode assemblies, such as example electrode assembly 200, can be removably received. As will be described more fully below, the electrode assembly 200 can be positionally adjustable within the electrode locations 112.

In some embodiments, the electrode assembly 200 includes a dowel 201, an electrode 202, corresponding electrode wires 114, and a port 204. The electrode 202 is coupled to one end portion of the dowel 201, and the port 204 is located at the other end portion of the dowel 201. The dowel 201 may include a passageway or tunnel to contain the electrode wires 144. In some embodiments, an electrical connector may be located on the surface of the dowel 201, and the electrode wires 144 can be connected to the electrode 202 via the connector. In some embodiments that include active electrodes, an electrode amplifier may also be contained within or attached to the dowel 201. However, in some embodiments, no such amplifier is included. It should be understood that the electrode assembly 200 is one non-limiting example of a type of electrode assembly that can be used with the EEG headsets provided herein, and the use of various other types of electrode assemblies are also within the scope of this disclosure. While the electrode assembly 200 is shown as extending from the rigid shell 111, such a configuration is not required. That is, in some embodiments the electrode assembly is flush or recessed in relation to the rigid shell 111.

In some embodiments, the dowel 201 comprises a non-conductive (e.g., plastic) material. In some such embodiments, in order to complete the Faraday cage, a conductive cap (not shown) can be installed over the electrode assembly 200 so that the electrode assembly 200 is contained below the conductive cap. The conductive cap can be in electrical communication with the EMI shield 124. In some such embodiments, the conductive cap can be hinged to the outer shell 111 and detainable (e.g., using a latch) in the orientation in which the electrode locations 112 are closed by the conductive cap. The conductive caps may be located at every electrode location 112 on the entire helmet 110, or only at selected locations in some embodiments.

In some embodiments, the dowel 201 comprises a conductive material. In some such embodiments, the conductive material of the dowel 201 can be in electrical communication with the EMI shield 124 so as to complete the Faraday cage. In some such embodiments, no aforementioned conductive cap is included. However, in some such embodiments the aforementioned conductive cap may also be included when the dowel 201 comprises a conductive material.

The electrode assembly 200 also includes the electrode 202. The electrode 202 is configured to make contact with the scalp of the user 12, to pick up EEG brain signals therefrom, and to transfer the signals to the wire 114. In some embodiments, the electrode 202 is spring loaded in relation to the dowel 201. In such embodiments, each electrode 202 thereby has an independent suspension by which it can conform to the local topography of the user's 12 scalp. In some embodiments, the electrode 202 is rigidly fixed to the end portion of the dowel 201.

In some embodiments, the electrode 202 can be configured to be used with a conductive fluidic medium (e.g., a gel, or liquid). In some such embodiments, the electrode 202 can be equipped with a through-hole that allows passage of the conductive fluidic medium therethrough, and to the skin of the user 12. In alternative embodiments, the electrode 202 can be configured to be used dry (without a conductive fluidic medium). In particular embodiments, a single EEG helmet 110 can be configured to use some dry electrodes 202 and some wet electrodes 202 (wet electrodes 202 are those that are configured to be used with the conductive fluidic medium).

The electrode assembly 200 also includes the port 204. The port 204 can be used to administer a supply of conductive fluidic medium to the electrode 202. In some embodiments, the port 204 may include a fitting, a pierce-able septum, or may be closeable using a replaceable plug or cap, and the like. In some implementations, a syringe is used to administer the supply of conductive fluidic medium via the port 204.

Such electrode assemblies 200 are individually positionable relative to helmet 110 to adapt to the particular head size and shape of the user 12. That is, the electrode assemblies 200 can be individually adjusted so that the electrodes 202 are conformed to the contoured topography of the user's 12 scalp. In that manner, accurate and repeatable pickup of EEG brain signals from multiple different and distributed surface locations on the user's 12 scalp is facilitated by the helmet 110.

Referring now to **FIG. 3C****,** a schematic depiction of an example design by which the electrode assemblies 200 can be individually positionable relative to the helmet 110 is provided. In this embodiment, the electrode assembly 200 is slidably coupled within a bore of a ball 220. The ball 220, in turn, is slidably and rotatably coupled within a socket 228 of the helmet 110. This ball 220 and socket 228 arrangement provides articulation of the electrode assembly 200 so that it can be adjusted in relation to the helmet 110 in multiple ways. For example, by sliding the electrode assembly 200 in relation to the bore of the ball 220, the electrode assembly can be raised or lowered. Also, by sliding the ball 220 in relation to the socket 228, the electrode assembly 200 can be translated in a two-dimensional plane. Further, by pivoting the ball 220 in relation to the socket 228, the electrode assembly 200 can be angulated. Such adjustability can be beneficial as to individually adapting each electrode assembly 200 to the particular head size and shape of the user 12. As a result, accurate and repeatable pickup of EEG brain signal information from multiple different and distributed surface locations on the user's 12 scalp is facilitated by the helmet 110.

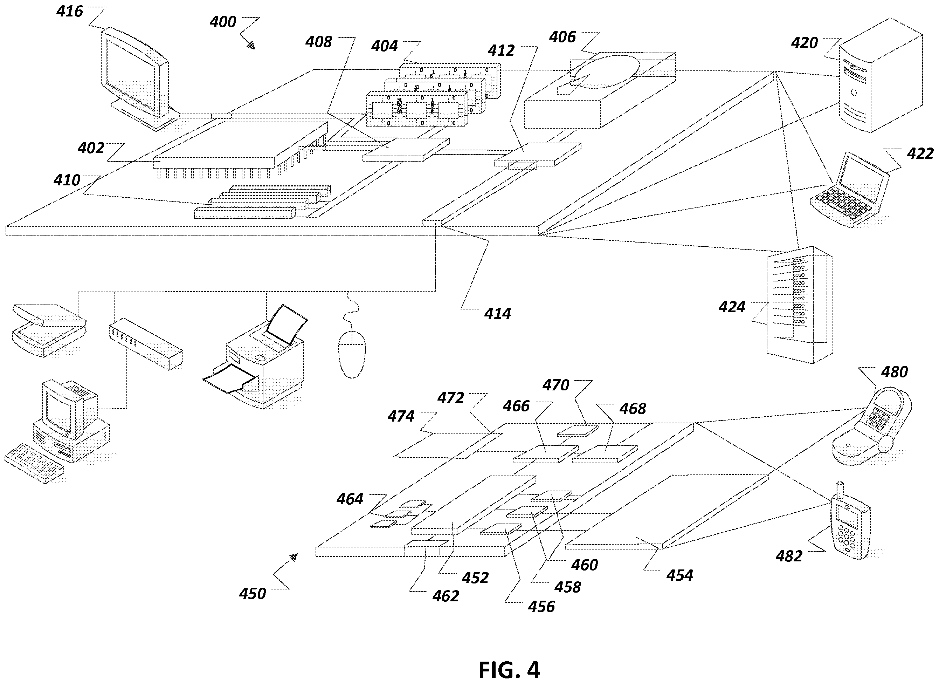

Referring now to **FIG. 4****,** an example of a computing device 400 and an example of a mobile computing device 450 that can be used to implement the techniques described herein are provided. The computing device 400 is intended to represent various forms of digital computers, such as laptops, desktops, workstations, personal digital assistants, servers, blade servers, mainframes, and other appropriate computers. The mobile computing device is intended to represent various forms of mobile devices, such as personal digital assistants, cellular telephones, smart-phones, and other similar computing devices. The components shown here, their connections and relationships, and their functions, are meant to be exemplary only, and are not meant to limit implementations of the inventions described and/or claimed in this document.

The computing device 400 includes a processor 402, a memory 404, a storage device 406, a high-speed interface 408 connecting to the memory 404 and multiple high-speed expansion ports 410, and a low-speed interface 412 connecting to a low-speed expansion port 414 and the storage device 406. Each of the processor 402, the memory 404, the storage device 406, the high-speed interface 408, the high-speed expansion ports 410, and the low-speed interface 412, are interconnected using various busses, and may be mounted on a common motherboard or in other manners as appropriate. The processor 402 can process instructions for execution within the computing device 400, including instructions stored in the memory 404 or on the storage device 406 to display graphical information for a GUI on an external input/output device, such as a display 416 coupled to the high-speed interface 408. In other implementations, multiple processors and/or multiple buses may be used, as appropriate, along with multiple memories and types of memory. Also, multiple computing devices may be connected, with each device providing portions of the necessary operations (e.g., as a server bank, a group of blade servers, or a multi-processor system).

The memory 404 stores information within the computing device 400. In some implementations, the memory 404 is a volatile memory unit or units. In some implementations, the memory 404 is a non-volatile memory unit or units. The memory 404 may also be another form of computer-readable medium, such as a magnetic or optical disk.

The storage device 406 is capable of providing mass storage for the computing device 400. In some implementations, the storage device 406 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The computer program product can also be tangibly embodied in a computer- or machine-readable medium, such as the memory 404, the storage device 406, or memory on the processor 402.

The high-speed interface 408 manages bandwidth-intensive operations for the computing device 400, while the low-speed interface 412 manages lower bandwidth-intensive operations. Such allocation of functions is exemplary only. In some implementations, the high-speed interface 408 is coupled to the memory 404, the display 416 (e.g., through a graphics processor or accelerator), and to the high-speed expansion ports 410, which may accept various expansion cards (not shown). In the implementation, the low-speed interface 412 is coupled to the storage device 406 and the low-speed expansion port 414. The low-speed expansion port 414, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 400 may be implemented in a number of different forms, as shown in the figure. For example, it may be implemented as a standard server 420, or multiple times in a group of such servers. In addition, it may be implemented in a personal computer such as a laptop computer 422. It may also be implemented as part of a rack server system 424. Alternatively, components from the computing device 400 may be combined with other components in a mobile device (not shown), such as a mobile computing device 450. Each of such devices may contain one or more of the computing device 400 and the mobile computing device 450, and an entire system may be made up of multiple computing devices communicating with each other.

The mobile computing device 450 includes a processor 452, a memory 464, an input/output device such as a display 454, a communication interface 466, and a transceiver 468, among other components. The mobile computing device 450 may also be provided with a storage device, such as a micro-drive or other device, to provide additional storage. Each of the processor 452, the memory 464, the display 454, the communication interface 466, and the transceiver 468, are interconnected using various buses, and several of the components may be mounted on a common motherboard or in other manners as appropriate.

The processor 452 can execute instructions within the mobile computing device 450, including instructions stored in the memory 464. The processor 452 may be implemented as a chipset of chips that include separate and multiple analog and digital processors. The processor 452 may provide, for example, for coordination of the other components of the mobile computing device 450, such as control of user interfaces, applications run by the mobile computing device 450, and wireless communication by the mobile computing device 450.

The processor 452 may communicate with a user through a control interface 458 and a display interface 456 coupled to the display 454. The display 454 may be, for example, a TFT (Thin-Film-Transistor Liquid Crystal Display) display or an OLED (Organic Light Emitting Diode) display, or other appropriate display technology. The display interface 456 may comprise appropriate circuitry for driving the display 454 to present graphical and other information to a user. The control interface 458 may receive commands from a user and convert them for submission to the processor 452. In addition, an external interface 462 may provide communication with the processor 452, so as to enable near area communication of the mobile computing device 450 with other devices. The external interface 462 may provide, for example, for wired communication in some implementations, or for wireless communication in other implementations, and multiple interfaces may also be used.

The memory 464 stores information within the mobile computing device 450. The memory 464 can be implemented as one or more of a computer-readable medium or media, a volatile memory unit or units, or a non-volatile memory unit or units. An expansion memory 474 may also be provided and connected to the mobile computing device 450 through an expansion interface 472, which may include, for example, a SIMM (Single In-Line Memory Module) card interface. The expansion memory 474 may provide extra storage space for the mobile computing device 450, or may also store applications or other information for the mobile computing device 450. Specifically, the expansion memory 474 may include instructions to carry out or supplement the processes described above, and may include secure information also. Thus, for example, the expansion memory 474 may be provide as a security module for the mobile computing device 450, and may be programmed with instructions that permit secure use of the mobile computing device 450. In addition, secure applications may be provided via the SIMM cards, along with additional information, such as placing identifying information on the SIMM card in a nonhackable manner.

The memory may include, for example, flash memory and/or NVRAM memory (non-volatile random access memory), as discussed below. In some implementations, a computer program product is tangibly embodied in an information carrier. The computer program product contains instructions that, when executed, perform one or more methods, such as those described above. The computer program product can be a computer- or machine-readable medium, such as the memory 464, the expansion memory 474, or memory on the processor 452. In some implementations, the computer program product can be received in a propagated signal, for example, over the transceiver 468 or the external interface 462.

The mobile computing device 450 may communicate wirelessly through the communication interface 466, which may include digital signal processing circuitry where necessary. The communication interface 466 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 468 using a radio-frequency. In addition, short-range communication may occur, such as using a Bluetooth, WiFi, or other such transceiver (not shown). In addition, a GPS (Global Positioning System) receiver module 470 may provide additional navigation-related and location-related wireless data to the mobile computing device 450, which may be used as appropriate by applications running on the mobile computing device 450.

The mobile computing device 450 may also communicate audibly using an audio codec 460, which may receive spoken information from a user and convert it to usable digital information. The audio codec 460 may likewise generate audible sound for a user, such as through a speaker, e.g., in a handset of the mobile computing device 450. Such sound may include sound from voice telephone calls, may include recorded sound (e.g., voice messages, music files, etc.) and may also include sound generated by applications operating on the mobile computing device 450.

The mobile computing device 450 may be implemented in a number of different forms, as shown in the figure. For example, it may be implemented as a cellular telephone 480. It may also be implemented as part of a smart-phone 482, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

Embodiments and all of the functional operations described in this specification may be implemented in digital electronic circuitry, or in computer software, firmware, or hardware, including the structures disclosed in this specification and their structural equivalents, or in combinations of one or more of them. Embodiments may be implemented as one or more computer program products, i.e., one or more modules of computer program instructions encoded on a computer readable medium for execution by, or to control the operation of, data processing apparatus. The computer readable medium may be a machine-readable storage device, a machine-readable storage substrate, a memory device, a composition of matter effecting a machine-readable propagated signal, or a combination of one or more of them. The term "data processing apparatus" encompasses all apparatus, devices, and machines for processing data, including by way of example a programmable processor, a computer, or multiple processors or computers. The apparatus may include, in addition to hardware, code that creates an execution environment for the computer program in question, e.g., code that constitutes processor firmware, a protocol stack, a database management system, an operating system, or a combination of one or more of them. A propagated signal is an artificially generated signal, e.g., a machine-generated electrical, optical, or electromagnetic signal that is generated to encode information for transmission to suitable receiver apparatus.

A computer program (also known as a program, software, software application, script, or code) may be written in any form of programming language, including compiled or interpreted languages, and it may be deployed in any form, including as a stand-alone program or as a module, component, subroutine, or other unit suitable for use in a computing environment. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform functions by operating on input data and generating output. The processes and logic flows may also be performed by, and apparatus may also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer may be embedded in another device, e.g., a tablet computer, a mobile telephone, a personal digital assistant (PDA), a mobile audio player, a Global Positioning System (GPS) receiver, to name just a few. Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory may be supplemented by, or incorporated in, special purpose logic circuitry.

While this specification contains many specifics, these should not be construed as limitations on the scope of the disclosure or of what may be claimed, but rather as descriptions of features specific to particular embodiments. Certain features that are described in this specification in the context of separate embodiments may also be implemented in combination in a single embodiment. Conversely, various features that are described in the context of a single embodiment may also be implemented in multiple embodiments separately or in any suitable subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described components and systems may generally be integrated together in a single product or multiple products.

A number of embodiments have been described. Nevertheless, it will be understood that various modifications may be made. In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. An apparatus for detecting brain signal information from a human' s head, the apparatus comprising:
a casing (110) including an outer surface and an inner surface, the casing's shape defining a concaved interior region configured to receive a portion of the head, the casing defining a plurality of electrode openings (112), each electrode opening of the plurality of electrode openings comprising a hole extending between the outer surface and the inner surface;
a conductive EMI shield material (124) disposed either i) on the inner or outer surfaces of the casing, ii) in a wall of the casing, or iii) within the interior region defined by the casing, the conductive EMI shield configured to operate as an EMI shield;
a conductive periphery (126) disposed around at least portions of an inner periphery of the casing, wherein the inner periphery of the casing interfaces with the skin of the human and the conductive periphery is in electrical communication with the EMI shield, and in physical contact with the skin of the human;
a conductive wire electrically coupled to the conductive EMI shield and configured to conduct energy received from the conductive EMI shield to a region of the human that is distinct from sites recording the brain signal information; and **characterised in that** the apparatus further comprises
a chin strap (120) coupled to the casing (110), wherein
a) the chin strap includes the conductive wire that is electrically coupled to the conductive EMI shield and a conductive contact element (122) that contacts the skin of the human beneath the chin
or
b) the chin strap is made of an electrically conductive material and is in electrical communication with the conductive EMI shield material (124) and the skin of the user.

2. The apparatus of claim 1, further comprising one or more electrode assemblies (200), wherein each electrode assembly of the one or more electrode assemblies is configured to be disposed within one electrode opening of the plurality of electrode openings, and wherein each electrode assembly is configured to receive the brain signal information from a surface of the human's head.

3. The apparatus of claim 2, wherein the one or more electrode assemblies are configured to be physically repositionable in relation to respective electrode openings of the casing.

4. That apparatus of claim 3, wherein the one or more electrode assemblies are configured to be slidably and pivotably repositionable in relation to respective electrode openings of the casing.

5. The apparatus of claim 1, further comprising controller circuitry including one or more microprocessors, wherein the controller circuitry is configured to receive the brain signal information.

6. The apparatus of claim 5, further comprising a user interface in electrical communication with the controller circuitry, wherein the user interface comprises one or more elements by which i) user inputs can be provided to the controller circuitry or ii) outputs can be provided from the controller circuitry.

7. The apparatus of claim 5, further comprising a communications interface in electrical communication with the controller circuitry, wherein the communications interface is configured to facilitate communications between the controller circuitry and an external computing device.

8. The apparatus of claim 7, wherein the communications interface is a wireless communications interface.

9. A brain-computer interface system (10) comprising:
a helmet-like apparatus for recording brain signal information from a human's head, the helmet-like apparatus comprising the apparatus for detecting brain signal information of any one of claims 1 to 8; and
a computing device (20) configured to receive communications from the helmet-like apparatus.

10. The system of claim 9, wherein the computing device is configured to process the captured brain signal information to detect if the captured brain signal information is indicative of an intention of the human.

11. The system of claim 10, wherein, in response to detecting that the captured brain signal information is indicative of an intention of the human, the computing device is configured to perform one or more actions corresponding to the intention.

12. The system of claim 9, wherein the communications comprise wireless communications.

## Patentansprüche

1. Einrichtung zum Detektieren von Gehirnsignalinformationen aus dem Kopf eines Menschen, wobei die Einrichtung Folgendes umfasst:
ein Gehäuse (110) mit einer äußeren Oberfläche und einer inneren Oberfläche, wobei die Form des Gehäuses einen konkaven Innenbereich definiert, der zur Aufnahme eines Teils des Kopfes ausgelegt ist, wobei das Gehäuse mehrere Elektrodenöffnungen (112) definiert, wobei jede Elektrodenöffnung der mehreren Elektrodenöffnungen ein Loch umfasst, das sich zwischen der äußeren Oberfläche und der inneren Oberfläche erstreckt;
ein leitfähiges EMI-Abschirmmaterial (124), das entweder i) auf der inneren oder äußeren Oberfläche des Gehäuses, ii) in einer Wand des Gehäuses oder iii) innerhalb des durch das Gehäuse definierten Innenbereichs angeordnet ist, wobei die leitfähige EMI-Abschirmung dazu ausgelegt ist, als EMI-Abschirmung zu wirken;
eine leitfähige Peripherie (126), die um zumindest Teile einer inneren Peripherie des Gehäuses angeordnet ist, wobei die innere Peripherie des Gehäuses mit der Haut des Menschen verbunden wird und sich die leitfähige Peripherie in elektrischer Kommunikation mit der EMI-Abschirmung und in physischem Kontakt mit der Haut des Menschen befindet;
einen leitfähigen Draht, der elektrisch mit der leitfähigen EMI-Abschirmung gekoppelt ist und dazu ausgelegt ist, Energie, die von der leitfähigen EMI-Abschirmung empfangen wird, an einen Bereich des Menschen zu leiten, bei dem es sich nicht um Stellen, an denen die Gehirnsignalinformationen aufgezeichnet werden, handelt;
und **dadurch gekennzeichnet, dass** die Einrichtung ferner Folgendes umfasst:
ein Kinnband (120), das mit dem Gehäuse (110) gekoppelt ist, wobei
a) das Kinnband den leitfähigen Draht, der elektrisch mit der leitfähigen EMI-Abschirmung gekoppelt ist, und ein leitfähiges Kontaktelement (122), das die Haut des Menschen unter dem Kinn berührt, aufweist,
oder
b) das Kinnband aus einem elektrisch leitfähigen Material besteht und mit dem leitfähigen EMI-Abschirmmaterial (124) und der Haut des Benutzers in elektrischer Kommunikation steht.

2. Einrichtung nach Anspruch 1, ferner umfassend eine oder mehrere Elektrodenbaugruppen (200), wobei jede Elektrodenbaugruppe der einen oder der mehreren Elektrodenbaugruppen dazu ausgelegt ist, innerhalb einer Elektrodenöffnung der mehreren Elektrodenöffnungen angeordnet zu werden, und wobei jede Elektrodenbaugruppe dazu ausgelegt ist, die Gehirnsignalinformationen von einer Oberfläche des Kopfes des Menschen zu empfangen.

3. Einrichtung nach Anspruch 2, wobei die eine oder die mehreren Elektrodenbaugruppen dazu ausgelegt sind, in Bezug auf die jeweiligen Elektrodenöffnungen des Gehäuses physisch neupositioniert zu werden.

4. Einrichtung nach Anspruch 3, wobei die eine oder die mehreren Elektrodenbaugruppen dazu ausgelegt sind, in Bezug auf die jeweiligen Elektrodenöffnungen des Gehäuses verschiebbar und drehbar neupositioniert zu werden.

5. Einrichtung nach Anspruch 1, ferner umfassend eine Steuerungsschaltungsanordnung mit einem oder mehreren Mikroprozessoren, wobei die Steuerungsschaltungsanordnung dazu ausgelegt ist, die Gehirnsignalinformationen zu empfangen.

6. Einrichtung nach Anspruch 5, ferner umfassend eine Benutzeroberfläche in elektrischer Kommunikation mit der Steuerungsschaltungsanordnung, wobei die Benutzeroberfläche ein oder mehrere Elemente umfasst, durch die i) der Steuerungsschaltungsanordnung Benutzereingaben bereitgestellt werden können oder ii) Ausgaben von der Steuerungsschaltungsanordnung bereitgestellt werden können.

7. Einrichtung nach Anspruch 5, ferner umfassend eine Kommunikationsschnittstelle in elektrischer Kommunikation mit der Steuerungsschaltungsanordnung, wobei die Kommunikationsschnittstelle dazu ausgelegt ist, die Kommunikation zwischen der Steuerungsschaltungsanordnung und einer externen Rechenvorrichtung zu ermöglichen.

8. Einrichtung nach Anspruch 7, wobei die Kommunikationsschnittstelle eine Drahtloskommunikationsschnittstelle ist.

9. Gehirn-Computer-Schnittstellensystem (10), das Folgendes umfasst:
eine helmartige Einrichtung zur Aufzeichnung von Gehirnsignalinformationen aus dem Kopf eines Menschen, wobei die helmartige Einrichtung die Einrichtung zum Detektieren von Gehirnsignalinformationen nach einem der Ansprüche 1 bis 8 umfasst; und
eine Rechenvorrichtung (20), ausgelegt zum Empfangen einer Kommunikation von der helmartigen Einrichtung.

10. System nach Anspruch 9, wobei die Rechenvorrichtung dazu ausgelegt ist, die erfassten Gehirnsignalinformationen zu verarbeiten, um zu detektieren, ob die erfassten Gehirnsignalinformationen auf eine Absicht des Menschen hinweisen.

11. System nach Anspruch 10, wobei die Rechenvorrichtung als Reaktion darauf, dass detektiert wird, dass die erfassten Gehirnsignalinformationen auf eine Absicht des Menschen hinweisen, dazu ausgelegt ist, eine oder mehrere Aktionen durchzuführen, die der Absicht entsprechen.

12. System nach Anspruch 9, wobei die Kommunikation eine drahtlose Kommunikation umfasst.

## Revendications

1. Appareil pour détecter des informations de signal cérébral en provenance de la tête d'un humain, l'appareil comprenant :
un boîtier (110) comprenant une surface extérieure et une surface intérieure, la forme du boîtier définissant une région intérieure concave configurée pour recevoir une partie de la tête, le boîtier définissant une pluralité d'ouvertures d'électrodes (112), chaque ouverture d'électrode de la pluralité d'ouvertures d'électrodes comprenant un trou s'étendant entre la surface extérieure et la surface intérieure ;
un matériau de blindage EMI conducteur (124) disposé soit i) sur les surfaces intérieures ou extérieures du boîtier, ii) dans une paroi du boîtier, ou iii) dans la région intérieure définie par le boîtier, le blindage EMI conducteur étant configuré pour fonctionner comme un blindage EMI ;
une périphérie conductrice (126) disposée autour d'au moins des parties d'une périphérie intérieure du boîtier, la périphérie intérieure du boîtier étant en interface avec la peau de l'humain et la périphérie conductrice étant en communication électrique avec le blindage EMI et en contact physique avec la peau de l'humain ;
un fil conducteur couplé électriquement au blindage EMI conducteur et configuré pour conduire l'énergie reçue en provenance du blindage EMI conducteur vers une région de l'humain qui est distincte de sites enregistrant les informations de signal cérébral ; et **caractérisé en ce que** l'appareil comprend en outre :
une mentonnière (120) couplée au boîtier (110),
a) la mentonnière comprenant le fil conducteur qui est couplé électriquement au blindage EMI conducteur à et un élément de contact conducteur (122) qui entre en contact avec la peau de l'humain sous le menton
ou
b) la mentonnière étant constituée d'un matériau électriquement conducteur et étant en communication électrique avec le matériau conducteur du blindage EMI (124) et la peau de l'utilisateur.

2. Appareil selon la revendication 1, comprenant en outre un ou plusieurs ensembles d'électrodes (200), chaque ensemble d'électrodes du ou des ensembles d'électrodes étant configuré pour être disposé dans une ouverture d'électrode de la pluralité d'ouvertures d'électrodes, et chaque ensemble d'électrodes étant configuré pour recevoir les informations de signal cérébral en provenance d'une surface de la tête d'humain.

3. Appareil selon la revendication 2, le ou les ensembles d'électrodes étant configurés pour être physiquement repositionnables par rapport aux ouvertures d'électrodes respectives du boîtier.

4. Appareil selon la revendication 3, le ou les ensembles d'électrodes étant configurés pour être repositionnables de manière coulissante et pivotante par rapport aux ouvertures d'électrodes respectives du boîtier.

5. Appareil selon la revendication 1, comprenant en outre un circuit de dispositif de commande comprenant un ou plusieurs microprocesseurs, le circuit de dispositif de commande étant configuré pour recevoir les informations de signal cérébral.

6. Appareil selon la revendication 5, comprenant en outre une interface utilisateur en communication électrique avec le circuit de dispositif de commande, l'interface utilisateur comprenant un ou plusieurs éléments par lesquels i) les entrées de l'utilisateur peuvent être fournies au circuit de dispositif de commande ou ii) les sorties peuvent être fournies à partir du circuit de dispositif de commande.

7. Appareil selon la revendication 5, comprenant en outre une interface de communication en communication électrique avec le circuit de dispositif de commande, l'interface de communication étant configurée pour faciliter les communications entre le circuit de dispositif de commande et un dispositif informatique externe.

8. Appareil selon la revendication 7, l'interface de communication étant une interface de communication sans fil.

9. Système d'interface cerveau-ordinateur (10) comprenant :
un appareil de type casque pour enregistrer des informations de signal cérébral provenant de la tête d'un humain, l'appareil de type casque comprenant l'appareil de détection d'informations de signal cérébral selon l'une quelconque des revendications 1 à 8 ; et
un dispositif informatique (20) configuré pour recevoir des communications de l'appareil de type casque.

10. Système selon la revendication 9, le dispositif informatique étant configuré pour traiter les informations de signal cérébral capturées pour détecter si les informations de signal cérébral capturées sont indicatives d'une intention de l'humain.

11. Système selon la revendication 10, en réponse à la détection que les informations de signal cérébral capturées sont indicatives d'une intention de l'humain, le dispositif informatique étant configuré pour effectuer une ou plusieurs actions correspondant à l'intention.

12. Système selon la revendication 9, les communications comprenant des communications sans fil.
